# EUROPÄISCHE PATENTANMELDUNG

(11) **EP 2 284 500 A1**
(43) Veröffentlichungstag der Anmeldung: **16.02.2011**
(21) Anmeldenummer: 09009914.4
(22) Anmeldetag: 31.07.2009
(51) Int. Cl.: G01F 5/00, A61B 5/00, A61J 13/00

(54) **Set umfassend ein Stillhütchen und eine Messvorrichtung zum Messen einer Milchmenge**

(71) Anmelder: Tritsch-Olian, Alexander, 53578 Windhagen (DE)
(72) Erfinder: Tritsch-Olian, Alexander, 53578 Windhagen (DE)
(74) Vertreter: Müller-Gerbes Wagner Albiger Patentanwälte

(57) **Zusammenfassung**

Die Erfindung betrifft eine Messvorrichtung zum Messen eines durch einen Druckunterschied bedingten Milchstroms durch einen Hohlkörper zu einem Säugling, wobei die Messvorrichtung umfasst: eine Messkammer zur Aufnahme eines Messstroms, eine Zuflussleitung zur Verbindung zwischen der Messkammer und dem Hohlkörper, eine Druckleitung zur Verbindung zwischen der Messkammer und dem Säugling sowie eine Fangvorrichtung, die in Einsatzlage der Messvorrichtung einen Austritt des in die Messkammer gelangten Messstroms verhindert. Die Messvorrichtung zeichnet sich dadurch aus, dass Befestigungsmittel vorgesehen sind, die eine lösbare Befestigung zwischen Messvorrichtung und Hohlkörper ermöglichen und dass die Messkammer und die Zuflussleitung unlösbar miteinander verbunden sind. Des Weiteren betrifft die Erfindung eine Set bestehend aus der Messvorrichtung und einem Stillhütchen sowie einem Verfahren zum Messen mit der Messvorrichtung bzw. mit dem Set.

## Beschreibung

Die Erfindung betrifft eine Messvorrichtung zum Messen eines durch einen Druckunterschied bedingten Fluidstroms durch einen Hohlkörper zu einem Verbraucher, insbesondere zum Messen einer Milchmenge, die ein Säugling aus einer Mutterbrust saugt. Die Erfindung betrifft auch ein Set, welches die Messvorrichtung und ein Stillhütchen umfasst. Des Weiteren betrifft die Erfindung ein Verfahren zum Messen mit der Messvorrichtung oder mit dem Set.

Aus der US 2006/0226108 A1 ist eine Messvorrichtung zum Messen einer Milchmenge bekannt, die ein Säugling aus einer Mutterbrust entnimmt. Die Milchmenge fließt dabei durch einen Hohlkörper, der durch einen im Wesentlichen kegelförmigen Saugnippel eines Stillhütchen begrenzt wird, welches an der Mutterbrust anliegt und dem Schutz der Brustwarze der Mutterbrust dient. Die Messvorrichtung umfasst eine Messkammer zur Aufnahme eines Messstroms, eine Zuflussleitung zur Verbindung zwischen der Messkammer und dem Hohlkörper sowie einer Druckleitung zur Verbindung zwischen der Messkammer und dem Säugling (Verbraucher), der Milch aus dem Hohlkörper saugt. Die Messvorrichtung weist des Weiteren eine Fangvorrichtung auf, die in Einsatzlage der Messvorrichtung einen Austritt des in die Messkammer gelangten Messstroms verhindert.

Wenn der Säugling an dem Saugnippel saugt, entsteht sowohl in der Messkammer als auch im Hohlkörper ein Unterdruck. Dieser Unterdruck zieht eine bestimmte Milchmenge in die Messkammer, wobei der die in die Messkammer gelangte Milchmenge (Messstrom) aufgrund der Fangvorrichtung in dieser verbleibt. Die Fluidmenge in der Messkammer ist dabei ein Maß für den Fluidstrom bzw. die Milchmenge, welcher bzw. welche der Säugling aus dem Hohlkörper entnommen hat. Soweit das Stillhütchen dicht an der Mutterbrust anliegt, entspricht die dem Hohlkörper entnommene Milchmenge der Milchmenge aus der Mutterbrust, abgesehen von der Milchmenge, die sich in der Messkammer befindet.

Die Verwendung der Messvorrichtung mit dem Stillhütchen gemäß der US 2006/0226108 A1 ist aus gesundheitlichen und hygienischen Gründen nicht unproblematisch. Nach Benutzung muss die Messkammer ausgewaschen werden, damit die in der Messkammer befindliche Milchmenge nicht verkrustet oder schimmelt. Das vollständige Auswaschen der Messkammer gestaltet sich dann besonders schwierig, wenn die Zuflussleitung, die Druckleitung und/oder die Messkammer sehr klein sind.

Bei der Messvorrichtung der US 2006/0226108 kann zudem das Problem auftreten, dass beim Stillen das Stillhütchen verformt wird und dadurch die Zuflussleitung und/oder Druckleitung ebenfalls deformiert werden. Wird beispielsweise dabei die Druckleitung abgequetscht, wird keine Milch mehr in die Messkammer gesaugt, was zu falschen Messergebnissen führt.

Der Erfindung liegt daher die Aufgabe zugrunde, eine Vorrichtung zum Messen eines Fluidstroms durch einen Hohlkörper zu einem Verbraucher, insbesondere zum Messen einer Milchmenge, die ein Säugling aus der Mutterbrust saugt, bereit zu stellen, durch die ein einfaches, kostengünstiges und unproblematisches und genaues Messen des Fluidstroms möglich ist.

Die der Erfindung zugrunde liegende Aufgabe wird mit der Messvorrichtung gemäß Anspruch 1 gelöst. Des Weiteren wird die Aufgabe mit der Bereitstellung eines Sets gemäß Anspruch 7 gelöst. Anspruch 15 schlägt ein Verfahren zum Messen eines Fluidstroms mit der erfindungsgemäßen Vorrichtung oder dem erfindungsgemäßen Set vor.

Die Messvorrichtung gemäß Anspruch 1 zeichnet sich dadurch aus, dass sie Befestigungsmittel umfasst, die eine lösbare Befestigung zwischen Messvorrichtung und Hohlkörper ermöglichen und dass die Messkammer und die Zuflussleitung unlösbar miteinander verbunden sind. Vorzugsweise sind auch die Druckleitung und auch die Fangvorrichtung unlösbar mit der Messkammer verbunden.

Somit bildet die Messvorrichtung eine Einheit, die lösbar mit dem Hohlkörper verbunden ist. Vorzugsweise umfassen die Befestigungsmittel eine lösbare Steckverbindung oder Teile einer solchen Steckverbindung, so dass die Messvorrichtung in einfacher Weise mit dem Hohlkörper verbunden werden kann. Die Befestigungsmittel können aber auch eine Schraubverbindung oder eine Rastverbindung umfassen. Durch die lösbare Verbindung ist es möglich, die Messvorrichtung von dem Hohlkörper zu trennen, was eine Reinigung der Messvorrichtung bzw. der Messkammer grundsätzlich erleichtert.

Vorzugsweise bilden Zuflussleitung, Druckleitung und Messkammer eine starre Einheit, die nicht oder nur bei sehr großen Kräften deformierbar ist. Dadurch wird gewährleistet, dass nicht durch Deformierungen falsche Messergebnisse zu befürchten sind (beispielsweise kann so keine Leitung abgequetscht werden).

In einem bevorzugten Ausführungsbeispiel ist die Messvorrichtung ein Spritzgrussteil aus Kunststoff. Vorzugsweise ist sie dabei einteilig, zweiteilig oder dreiteilig ausgebildet, wobei in einem bevorzugten Ausführungsbeispiel Messkammer, Zuflussleitung, Druckleitung und Fangvorrichtung einstückig ausgebildet sind. Ein zweites Teil kann ein Deckel sein, der die Messkammer abschließt und mit dem anderen Teil in geeigneter Weise verbunden ist. Dadurch lassen sich die Produktionskosten für die Messvorrichtung derart günstig gestalten, dass ein nur einmaliger Gebrauch in Betracht kommt. Dies hat den Vorteil, dass die Messkammer nach erfolgter Messung nicht mehr ausgespült werden muss oder entleert werden muss. Somit kann, unabhängig von der Ausführung als Spritzgussteil, die Messvorrichtung besonders einfach aufgebaut sein, da die Messkammer zum Reinigen nicht geöffnet werden muss. Entsprechend ist die Messvorrichtung mit einer Messkammer ausgestattet, welche abgesehen von Druckleitung und Zuflussleitung keine Öffnung oder keine Öffnungsmöglichkeit aufweist.

Zudem kann die Messvorrichtung einen Einsatz wie beispielsweise eine Kalibriernadel aufweisen, welcher in eine Leitung gesetzt wird und durch den die Größe des verbleibenden Strömungsquerschnitts der Leitung genau eingestellt werden kann.

Vorzugsweise weist die Fangvorrichtung wenigstens eine Rohröffnung auf, welche in Einsatzlage der Messvorrichtung sich oberhalb eines Flüssigkeitsspiegels in der Messkammer befindet. Die oberhalb des Flüssigkeitsspiegels befindliche Rohröffnung kann dabei ein Ende der Zuflussleitung oder ein Anfang der Druckleitung sein. Ist die Rohröffnung der Anfang der Druckleitung, durch die der Unterdruck in der Messkammer aufgebaut wird, wird lediglich Luft aus der Messkammer und nicht die in die Messkammer gelangte Milchmenge bzw. der in die Messkammer gelangte Messstrom abgesaugt.

Die Messkammer kann eine Skala zum Ablesen des in der Messkammer gelangten Messstroms aufweisen. Alternativ kann die Skala auch die Menge des Fluidstroms ausweisen, der durch den Hohlkörper zum Verbraucher gelangt ist. Hierzu muss das Verhältnis von Messstrom und Fluidstrom bekannt sein. Das Verhältnis hängt dabei von der Gestaltung und Aufbau der Messvorrichtung ab (beispielsweise das Verhältnis eines Strömungsquerschnitts der Zuflussleitung zum Strömungsquerschnitt, durch den der Fluidstrom zum Verbraucher gelangt).

Damit der in die Messkammer gelangte Messstrom bezogen auf den Fluidstrom klein ist, ist vorzugsweise der Strömungsquerschnitt der Zuflussleitung klein. Die Zuflussleitung und/oder die Druckleitung können somit Kapillare sein. In einem bevorzugten Ausführungsbeispiel ist die Messvorrichtung derart gestaltet, dass das Verhältnis von Messstrom zum Fluidstrom, der zum Verbraucher gelangt, kleiner als ein 1/20 oder ein 1/50. In einem besonders bevorzugten Ausführungsbeispiel ist das Verhältnis kleiner als ein 1/100.

Die Messvorrichtung kann im Zusammenhang mit verschiedensten Hohlkörpern eingesetzt werden. Beispielsweise kann der Hohlkörper ein Trinkstrohhalm sein, auf den die Messrichtung aufgesteckt oder aufgeschoben werden kann. Die Messvorrichtung weist dabei an einem dem Trinkstrohhalm abgewandten Ende ein rohrförmiges Ende auf, an dem ein Verbraucher saugen kann.

Der Hohlkörper kann jedoch auch eine Flasche, Behälter oder dergleichen sein, wobei die Messvorrichtung in der Flasche oder an einer Öffnung der Flasche lösbar befestigt ist. Die Messvorrichtung misst dabei die Menge, die der Flasche oder dem Behälter entnommen wird.

Ein bevorzugtes Einsatzgebiet für die Messvorrichtung ist der Ausdauersport, bei dem beispielsweise ein auf dem Fahrrad sitzender Triathlet oder Radfahrer ständig Flüssigkeit aus einem Behälter entnehmen muss, den er als Rucksack auf dem Rücken trägt. Durch eine Saugleitung, an der die Messvorrichtung befestigt ist, kann der Radfahrer die Flüssigkeit mit seinem Mund ansaugen. Die Messvorrichtung ist dabei so angeordnet, dass es dem Radfahrer möglich ist, beim Fahrradfahren die in die Messkammer gelangte Flüssigkeitsmenge bzw, entsprechend umgerechnet und auf einer Skala berücksichtigt, die getrunkene Flüssigkeitsmenge abzulesen. Damit kann der Radfahrer seine Flüssigkeitszunahme genau kontrollieren und steuern.

Vergleichbare Anwendungen für die Messvorrichtung liegen beim Militär (beispielsweise die Flüssigkeitszunahme von Soldaten in Wüstengegenden), im Gesundheitswesen oder in der Betreuung von Behinderten oder älteren Menschen. Grundsätzlich lässt sich die Messvorrichtung immer dort einsetzen, wo ein Fluidstrom in einfacher Weise gemessen werden muss. Auch ist es denkbar, die Messvorrichtung mit Mitteln zu versehen, die ein Signal erzeugen, wenn die Messkammer einen bestimmten Flüssigkeitsspiegel aufweist. Das erzeugte Signal kann dann dafür benutzt werden, einen Schalter oder ähnliches anzusteuern, um den Fluidstrom im Hohlkörper zu unterbrechen oder um eine andere Aktion einzuleiten. Beispielsweise könnte eine derart ausgestattete Messvorrichtung im Haushalt, bei der Bewässerung, in Baumschulen und Privatgärten, bei Wohnmobilduschen, Duschen auf dem Campingplatz, am Strand im Bad etc. eingesetzt werden.

Eine besonders bevorzugte Anwendung liegt jedoch in Bestimmung der Milchmenge, die ein Säugling der Mutterbrust entnimmt. Es wird daher gemäß Anspruch 7 ein Set vorgeschlagen, das eine Messvorrichtung, wie sie in ihren unterschiedlichen Ausführungsbeispielen oben beschrieben wird, und ein Stillhütchen umfasst. Das Stillhütchen weist dabei einen Rand zur Anlage an der Mutterbrust und einen im Wesentlichen kegelförmigen Saugnippel auf, der dem Schutz der Brustwarze der Mutterbrust dient und in angelegter Position einen Hohlraum zwischen Brustwarze und Stillhütchen begrenzt. Das Set zeichnet sich dadurch aus, dass die Messvorrichtung in dem Hohlraum, also innerhalb des Saugnippels, angeordnet ist und lösbar mit dem Stillhütchen verbunden ist. Durch die Lösbarkeit kann die Messvorrichtung jederzeit vom Stillhütchen getrennt werden.

Vorzugsweise weist die Messvorrichtung eine gewölbte Oberseite und eine Unterseite auf, wobei die Oberseite an einer Innenseite des Saugnippels anliegt und die Unterseite zum verbleibenden Hohlraum zeigt. Die Messvorrichtung passt sich aufgrund der gewölbten Oberseite der Form des Saugnippels an, wobei genügend Raum innerhalb des Saugnippels zur Aufnahme der Brustwarze und zur Ausbildung des Hohlraums verbleibt.

Die Oberseite der Messvorrichtung kann dabei die Messkammer begrenzen. An einem Anlagebereich, an der die Oberseite der Messvorrichtung anliegt, kann der Saugnippel eine Skala für den in die Messkammer gelangten Messstrom bzw., unter Berücksichtigung der entsprechenden Verhältnisse, für die getrunkene Milchmenge aufweisen. Damit ist es nicht erforderlich, eine Skala auf der Messvorrichtung aufzubringen, was deren Herstellung weiter vereinfacht. Der Flüssigkeitsspiegel in der Messkammer muss dabei durch den Saugnippel erkennbar sein. Vorzugsweise ist daher der Saugnippel aus transparenten Material.

Der Rand des Stillhütchens kann eine Markierung zur richtigen Ausrichtung des Sets tragen. Durch die richtige Ausrichtung des Sets kann sichergestellt werden, dass beim Stillen des Säuglings keine Milchmenge aus der Messkammer austreten kann.

An dem Anlagebereich des Saugnippels kann wenigstens eine rohrförmige Leitung vorgesehen sein, die sich vom Hohlraum durch den Saugnippel (durch die Saugnippelwandung) nach außen zu einer Spitze des Saugnippels erstreckt und in Verbindung zur Druckleitung der Messvorrichtung steht. Durch diese rohrförmige Leitung durch den Saugnippel hindurch wird sichergestellt, dass die Messkammer mit Unterdruck beaufschlagt wird, die durch das Saugen oder Nuckeln des Säuglings entsteht. Der Säugling umschließt dabei mit seinem Mund von außen zumindest die Spitze des Saugnippels, so dass der im Säuglingsmund herrschende Unterdruck auch auf die Messkammer wirkt.

In einem bevorzugten Ausführungsbeispiel ist an der Oberseite der Messvorrichtung wenigstens eine nutförmige Vertiefung zur Aufnahme der rohrförmigen Leitung des Saugnippels vorgesehen. In die Vertiefung, welche vorzugweise im Wesentlichen geradlinig ist und eine Längsachse aufweist, kann sich ein Rohrende der Druckleitung erstrecken. Das Rohrende und die Vertiefung weisen dabei bevorzugt parallele Längsachse auf. Die Messvorrichtung ist dabei durch Stecken des Rohrendes in die rohrförmige Leitung an dem Saugnippel bzw. an dem Stillhütchen befestigbar ist. In einem bevorzugten Ausführungsbeispiel sind zwei parallele Vertiefungen mit jeweils einem Rohrende der Druckleitung vorgesehen. Es ist aber auch möglich, nur eine Vertiefung oder auch mehrere Vertiefungen und entsprechend viele rohrförmige Leitungen vorzusehen.

Die Messvorrichtung kann einen Durchströmkanal aufweisen, welcher durch einen Mittelsteg geteilt ist, in dem die Zuflussleitung eingearbeitet ist. Der Durchströmkanal ist dabei in der Nähe der Spitze des Saugnippels angeordnet, wobei durch an der Spitze des Saugnippels angeordnete Öffnungen die Muttermilch in den Säuglingsmund gelangt. Durch die im Durchströmkanal angeordnete Zuflussleitung lassen sich besonders genaue Messergebnisse erzeugen.

Das Verfahren gemäß Anspruch 15 zum Messen eines Fluidstroms mit einer oben beschriebenen Messvorrichtung oder mit einem oben beschriebenen Set zeichnet sich dadurch aus, dass die Messvorrichtung als Einwegartikel eingesetzt wird. Ist die Messkammer einmal voll gelaufen, so wird die Messvorrichtung zu Messzwecken nicht mehr eingesetzt. Die Messvorrichtung kann jedoch aufbewahrt werden, um zu einem späteren Zeitpunkt den vom Verbraucher aufgenommenen Fluidstrom in Abhängigkeit der Zeit nachvollziehen zu können. Beispielsweise könnte die stillende Mutter nach jedem Stillen die Messvorrichtung mit der vollgelaufenen Messkammer aufbewahren, um so ein Bild zu erlangen, wie viel und wann der Säugling Muttermilch in den letzten Tagen zu sich genommen hat.

Anhand der in der Zeichnung dargestellten Ausführungsbeispiele wird die Erfindung näher erläutert. Es zeigen:
- Figur 1: schematisch eine Messvorrichtung mit einem Hohlkörper;
- Figur 2: eine Messvorrichtung mit einem Stillhütchen in Einsatzlage;
- Figur 3: diverse Ansichten der Messvorrichtung der Figur 2 (Figuren 3a - 3e, wobei Figur 3e zusätzlich einen Deckel zeigt);
- Figur 4: diverse Ansichten des Stillhütchens mit Messvorrichtung gemäß Figur 2 (siehe Figuren 4a -4d);
- Figur 5: perspektivische Ansicht ein weiteres Ausführungsbeispiel für die Messvorrichtung;
- Figur 6: diverse Ansichten des Ausführungsbeispiels der Figur 5 (siehe Figuren 6a - 6e) und
- Figur 7: diverse Ansichten eines weiteren Ausführungsbeispiels (Fi- gur 7a -7d.

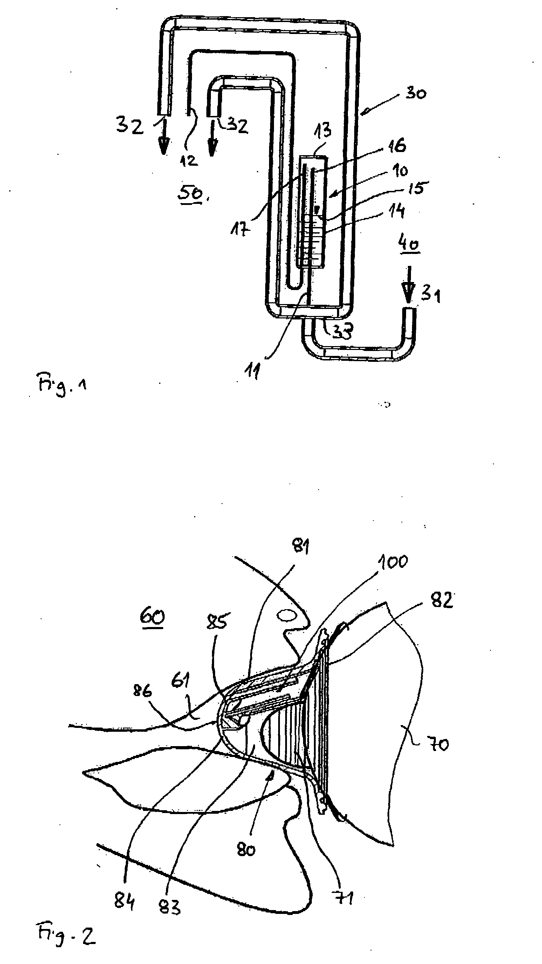

Figur 1 zeigt in schematischer Weise eine Messvorrichtung, die in ihrer Gesamtheit mit 10 bezeichnet wird. Zu beachten ist, dass anhand Figur 1 lediglich die Funktionsweise der Messvorrichtung 10 verdeutlicht werden soll.

Die Messvorrichtung 10 weist eine Zuflussleitung 11, eine Druckleitung 12 und eine Messkammer 13 auf. Figur 1 zeigt des Weiteren ein Rohrsystem 30 mit einem Eingang 31 und zwei Ausgängen 32. An einem Verteilungs- oder T-Stück 33 des Rohrsystems 30 ist die Messvorrichtung 10 über die Zuflussleitung 11 an dem Rohrsystem 30 angeschlossen. Das Rohrsystem 30 ist an dem Eingang 31 mit einer hier nicht dargestellten Flüssigkeitsquelle 40 verbunden. An den Ausgängen 32 ist das Rohrsystem 30 mit einem Verbraucher 50 verbunden. Des Weiteren ist auch die Druckleitung 12 mit dem Verbraucher 50 verbunden. Wird nun durch den Verbraucher 50 ein Unterdruck im Rohrsystem 30 und auch in der Druckleitung 12 angelegt, fließt ein Fluid (beispielsweise eine Flüssigkeit) von der Quelle 40 durch das Rohrsystem 30 zum Verbraucher 50. Da auch die Messvorrichtung 10 über die Druckleitung 12 mit dem Unterdruck beaufschlagt wird, gelangt dabei ein gewisser Anteil der Flüssigkeitsmenge durch die Zuflussleitung 11 in die Messkammer 13. Eine Skala 14, die durch Striche auf der Messkammer 13 angedeutet ist, kann so skaliert sein, dass ein Flüssigkeitsspiegel 15 in der Messkammer 13 die Flüssigkeitsmenge angibt, die durch das Rohrsystem 30 zum Verbraucher gelangt ist. Bei der Skalierung der Skala 14 müssen dabei verschiedenste Faktoren berücksichtigt werden, von denen das Verhältnis der in die Messkammer 13 gelangten Flüssigkeitsmenge und die zum Verbraucher gelangte Flüssigkeitsmenge abhängt. Eine Möglichkeit zur Bestimmung des Verhältnisses besteht durch eine erstmalige Kalibrierung, bei der die zum Verbraucher gelangte Flüssigkeitsmenge durch eine andere Messmethode bestimmt wird und ins Verhältnis gesetzt wird zu der Menge, die sich bei der erstmaligen Kalibrierung in der Messkammer befindet.

Die Zuflussleitung 11 ist als eine Kapillare mit einem oberen Rohrende 16 ausgebildet. Ein in der Messkammer befindliches Rohrende 17 der Druckleitung 12 liegt auch oberhalb des Flüssigkeitsspiegels 15 in der Messkammer. Durch die Position des Rohrendes 17 oberhalb der in der Messkammer befindlichen Flüssigkeitsmenge wird sichergestellt, dass keine Flüssigkeitsmenge aus der Messkammer 13 zum Verbraucher 50 gelangt und somit das Messergebnis verfälschen würde.

Figur 2 zeigt schematisch in Teilen den Kopf eines Säuglings 60, der aus einer Mutterbrust 70 gestillt wird. In dem Mund 61 des Säuglings und der Mutterbrust 70 ist ein Stillhütchen 80 mit einem Saugnippel 81 angeordnet, der zum Schutz der Brustwarze 71 der Mutterbrust 70 dient. Innerhalb des Saugnippels 81 ist eine Messkammer 100 angeordnet. Das Stillhütchen 80 und die Messvorrichtung 100 werden in den Figuren 2 bis 4 näher dargestellt.

Ein Rand 82 des Stillhütchens 80 liegt an der Mutterbrust 70 dichtend an, so dass sich ein abgeschlossener Hohlraum 83 zwischen dem Saugnippel 81 und der Mutterbrust 70 ausbildet. In diesem Hohlraum 83 befindet sich die Messkammer 100 und die Brustwarze 71 der Mutterbrust 70. An einer Spitze 86 des Saugnippels 81 sind Öffnungen 84, 85 vorgesehen. Die Öffnungen 84, 85 können jeweils mehrere Teilöffnungen aufweisen.

Saugt nun der Säugling 60 an dem Saugnippel 81, entsteht im Hohlraum 83 ein Unterdruck, durch den Milch aus der Mutterbrust 70 angesaugt wird. Diese Milch gelangt dann in den Mund 61 des Säuglings 60. Mit dem Saugen wird gleichzeitig in der Messvorrichtung 100 über die Öffnung 85 ein Unterdruck erzeugt, so dass ein sehr kleiner Teil der aus der Mutterbrust entnommenen Milch in die Messvorrichtung 100 gelangt. Die in die Messvorrichtung gelangte Milchmenge lässt, wie oben bereits dargelegt, einen Rückschluss auf die insgesamt der Mutterbrust entnommenen Milch zu.

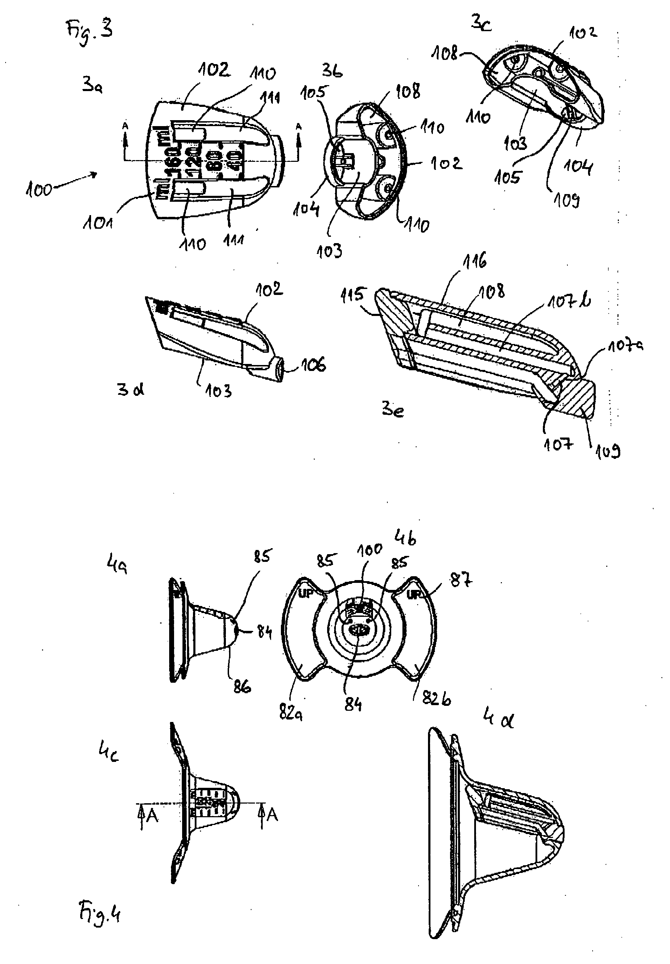

Figuren 3 und 4 zeigen die Messvorrichtung 100 und das Stillhütchen 80 in verschiedenen. Ansichten. Beispielsweise zeigt Figur 4c das Stillhütchen 80 von oben. Zu erkennen ist dabei eine Skala 101, die auf einer Oberseite 102 der Messvorrichtung 100 vorgesehen ist. Die Skala 101 ist durch das durchsichtige Silikonmaterial des Stillhütchens 80 sichtbar. In den Figuren 4a bis 4d sind die Öffnung 84 und die darüber angeordneten Öffnungen 85 an der Spitze 86 des Saugnippels 81 zu erkennen. Der Rand 82 besteht aus zwei Randsegmenten 82a, 82b, wobei jeweils auf jedem Randsegment 82a, 82b eine Markierung 87 angebracht ist, die dazu dient, das Stillhütchen 80 richtig auszurichten. Eine richtige Ausrichtung ist dann gegeben, wenn sich die ebenfalls in Figur 4 zu erkennende Messvorrichtung 100 oben (d.h. bei 12.00 Uhr) befindet.

Wie insbesondere den Figuren 3b und 3c zu entnehmen ist, ist die Oberseite 102 der Messvorrichtung 100 gewölbt ausgebildet, so dass sie sich dem im Wesentlichen kegelförmigen Stillhütchen 81 an dessen ebenfalls gewölbter Innenseite gut anlegen lässt.

An einer Unterseite -103 der Messvorrichtung 100 ist ein Bügel 104 angeordnet, der einen Durchströmkanal 105 der Messvorrichtung 100 definiert. Der Durchströmkanal 105 liegt mit einem äußeren Ende 106 direkt an der Öffnung 84 des Stillhütchens 80 an. Durch diesen Durchströmkanal 105 wird die gesamte Milchmenge geführt, die der Mutterbrust 70 durch das Saugen des Säuglings 60 entnommen wird. Ein sehr kleiner Teilstrom gelangt dabei durch eine Zuflussleitung 107 (siehe Figur 3e) in eine Messkammer 108. Die Zuflussleitung 107 ist dabei in einem Mittelsteg 109 eingearbeitet, der den Durchströmkanal 105 in zwei Teilkanäle aufteilt.

Die Zuflussleitung 107 besteht dabei aus einem ersten Leitungsstück 107a durch den Mittelsteg und einem Rohrstück 107b, welches in der Messkammer 108 angeordnet ist. Durch die Rohrabschnitte 107a, 107b gelangt Milch in die Messkammer 108.

Wie insbesondere Figur 3a zu entnehmen ist, sind an der Oberseite 102 zwei Druckleitungen 110 angeformt, die sich in zwei parallel zueinander ausgerichteten Vertiefungen 111 in der Oberseite 102 erstrecken. Die Vertiefungen 111 nehmen in Einsatzlage der Messvorrichtung 100 rohrförmige, in die Saugnippelwandung des Saugnippels eingearbeitete Leitungen auf, die in einem Anlagebereich angeordnet sind, an der die Oberseite 102 der Messvorrichtung 100 am Saugnippel 80 anliegt. Diese vorzugsweise einstückig an dem Saugnippel angeformte röhrförmigen Leitungen bilden eine Verbindung der Druckleitungen 110 zu den Öffnungen 85 des Saugnippels 80. Die Druckleitungen 110 lassen sich dabei in die in den Saugnippel 80 eingearbeitete Leitungen einschieben, sodass die Druckleitungen 110 inwandig und die Vertiefungen 111 auswandig an den Leitungen anliegen. Dadurch ist eine einfache Steckverbindung zwischen Messvorrichtung 100 und Stillhütchen 80 gegeben, welche sich einfach lösen lässt.

Saugt nun der Säugling 60 an dem Saugnippel 80, wird die Messkammer 100 über die Öffnungen 84 und die Druckleitungen 110 mit einem Unterdruck beaufschlagt, der dem Unterdruck entspricht, mit dem durch die Öffnung 84 auch der Hohlraum 83 innerhalb des Saugnippels 81 beaufschlagt wird. Beim Saugen des Säuglings 60 gelangt somit nicht nur Milch durch die Öffnung 84, sondern auch durch die Zuflussleitung 107 in die Messkammer 108.

Figur 3e zeigt im Schnitt die Messvorrichtung 100 mit einem Deckel 115, der die Messkammer 108 abschließt. Der Deckel 115 ist mit einem einstükigen Gehäuse 116 der Messvorrichtung 100 luftdicht verbunden. Somit lässt sich die Messvorrichtung durch lediglich zwei Spritzgussteile aus Kunststoff herstellen. Zu betonen ist, dass Messkammer 108, Zuflussleitung 107 und Druckleitung 110 somit aus einem Stück sind.

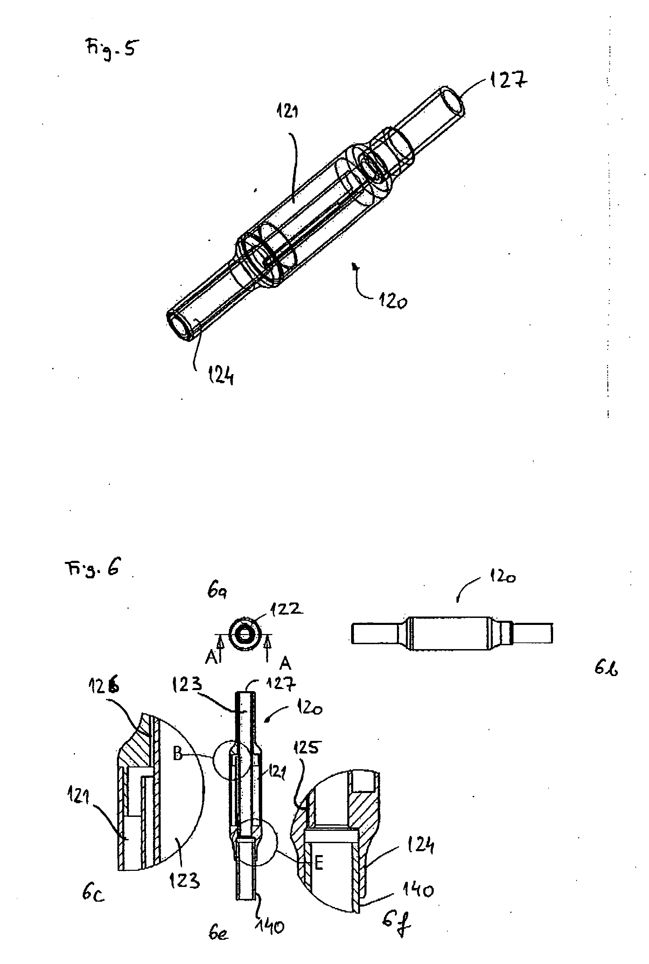

Die Figuren 5 und 6 zeigen ein weiteres Ausführungsbeispiel für eine Messkammer, die nun in ihrer Gesamtheit mit 120 bezeichnet wird. Die Messvorrichtung 120 ist im Wesentlichen rotationssymmetrisch aufgebaut und weist eine zylindrische Messkammer 121 mit scheibenförmigem Querschnitt 122 (siehe Figur 6a) auf. Die Messkammer 122 umschließt einen Durchströmkanal 123.

Die Messvorrichtung 120 weist ein ringförmiges Ende 124 auf, das einen nur ausschnittsweise dargestellten Trinkstrohhalm 140 an seinem Ende umgreifen kann. Die Messvorrichtung 120 weist zudem eine Zuflussleitung 125 auf, durch die ein gewisser Teilstrom der durch den Strohhalm 140 fließenden Flüssigkeitsmenge in die Messkammer 121 gelangt. Wird an einem oberen Ende 127 der Messvorrichtung gesaugt, entsteht aufgrund einer Druckleitung 126, welche sich bis zum oberen Ende 127 erstreckt, in der Messkammer 121 ein Unterdruck, durch den ein gewisser Teilstrom in die Messkammer 121 gelangt. Beim Saugen am oberen Ende 127 füllt sich die Messkammer 121 kontinuierlich auf. Die Flüssigkeitsmenge in der Messkammer 121 ist dabei proportional zu der insgesamt durch den Trinkstrohhalm geführten Flüssigkeitsmenge.

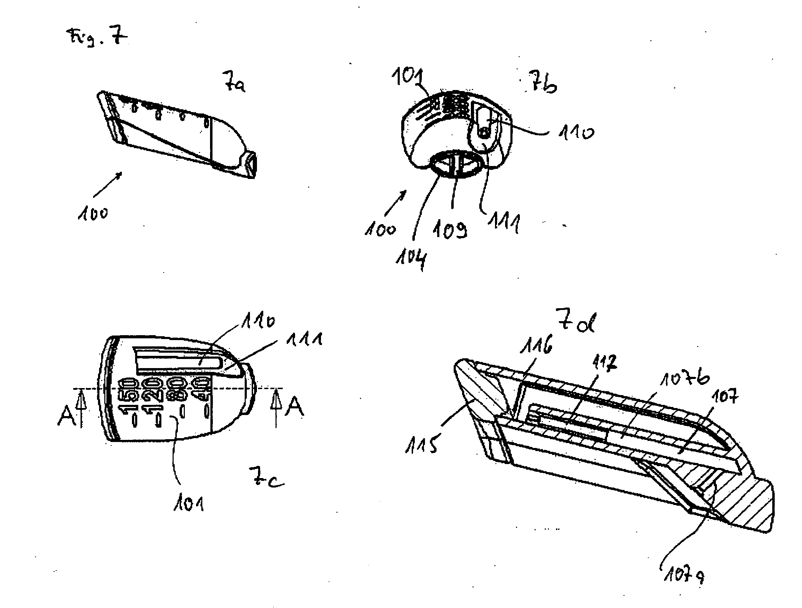

Ein weiteres Ausführungsbeispiel zeigen die Figuren 7a bis 7d. Im Gegensatz zum Ausführungsbeispiel der Figur 3 weist die hier dargestellte Messkammer 100 nur eine Druckleitung 110 und entsprechend auch nur eine Vertiefung 111 auf. Entsprechend würde das dazu korrespondierende Stillhütchen auch nur eine rohrförmige Leitung aufweisen.

Die Druckleitung 110 erstreckt sich zudem fast über die gesamte Länge der Vertiefung 111. Eine Skala 101 ist nun nicht mehr, wie im Ausführungsbeispiel der Figur 3, mittig angeordnet, sondern seitlich versetzt.

Ein weiteres Abgrenzungsmerkmal zum Ausführungsbeispiel der Figur 3 ist in Figur 7d zu erkennen, welches ein Schnitt entlang der Linie A-A der Figur 7c ist. In dem Teil 107b der Zuflussleitung 107 ist eine Kalibriernadel 117 angeordnet, welche den Strömungsquerschnitt der Zuflussleitung 107b entsprechend reduziert. Es entsteht somit ein ringförmiger Strömungsquerschnitt, der über die Kalibriernadel 112 in seiner Größe genau eingestellt werden kann. Über den Deckel 115 ist sichergestellt, dass die Kalibriernadel nicht aus der Zuflussleitung 107b heraus fällt.

Die Kalibriernadel 117 ist vorzugsweise aus einem anderen Material wie das Gehäuse 116. Vorzugsweise ist die Kalibriernadel aus Metall, sie kann aber auch aus einem Kunststoff sein.

Der Vollständigkeit halber wird darauf hingewiesen, dass die Kalibriernadel 117 auch bei Ausführungsbeispiel der Figur 3 eingesetzt werden kann, welches zwei Druckleitungen 110 aufweist.

### Bezugszeichenliste

- 10: Messvorrichtung
- 11: Zuflussleitung
- 12: Druckleitung
- 13: Messkammer
- 14: Skala
- 15: Flüssigkeitsspiegel
- 16: Rohrende
- 17: Rohrende
- 30: Rohrsystem
- 31: Einlass
- 32: Auslass
- 33: T-Stück
- 40: Quelle
- 50: Verbraucher
- 60: Säugling
- 61: Mund
- 70: Mutterbrust
- 71: Brustwarze
- 80: Stillhütchen
- 81: Saugnippel
- 82: Rand (82a, 82b)
- 83: Hohlraum
- 84: Öffnung
- 85: Öffnung
- 86: Spitze
- 87: Markierung
- 100: Messvorrichtung
- 101: Skala
- 102: Oberseite
- 103: Unterseite
- 104: Bügel
- 105: Durchflusskanal
- 106: Ende
- 107: Zuflussleitung (107a, 107b)
- 108: Messkammer
- 109: Mittelsteg
- 110: Druckleitung
- 111: Vertiefung
- 115: Deckel
- 116: Gehäuse
- 117: Kalibriernadel
- 120: Messvorrichtung
- 121: Messkammer
- 122: scheibenförmiger Querschnitt
- 123: Durchflussleitung
- 124: ringförmiges Ende
- 125: Zuflussleitung
- 126: Druckleitung
- 127: oberes Ende
- 140: Trinkstrohhalm

## Patentansprüche

1. Messvorrichtung zum Messen eines durch einen Druckunterschied bedingten Fluidstroms durch einen Hohlkörper zu einem Verbraucher, wobei die Messvorrichtung umfasst:
eine Messkammer zur Aufnahme eines Messstroms,
eine Zuflussleitung zur Verbindung zwischen der Messkammer und dem Hohlkörper
eine Druckleitung zur Verbindung zwischen der Messkammer und dem Verbraucher,
eine Fangvorrichtung, die in Einsatzlage der Messvorrichtung einen Austritt des in die Messkammer gelangten Messstroms verhindert,
**dadurch gekennzeichnet, dass** Befestigungsmittel vorgesehen sind, die eine lösbare Befestigung zwischen Messvorrichtung und Hohlkörper ermöglichen und dass die Messkammer und die Zuflussleitung unlösbar miteinander verbunden sind.

2. Messvorrichtung nach Anspruch 1, **dadurch gekennzeichnet, dass** die Befestigungsmittel eine lösbare Steckverbindung oder Teile einer solchen Steckverbindung umfassen.

3. Messvorrichtung nach Anspruch 1 oder 2, **dadurch gekennzeichnet, dass** die Messvorrichtung ein einteiliges oder zweiteiliges Spritzgussteil aus Kunststoff ist.

4. Messvorrichtung nach einem der Ansprüche 1 bis 3, **dadurch gekennzeichnet, dass** die Fangvorrichtung wenigstens eine Rohröffnung aufweist, welche in Einsatzlage der Messvorrichtung sich oberhalb eines Flüssigkeitsspiegels in der Messkammer befindet.

5. Messvorrichtung nach einem der Ansprüche 1 bis 4, dass die Messkammer eine Skala zum Ablesen des in der Messkammer gelangten Messstroms aufweist

6. Messvorrichtung nach einem der Ansprüche 1 bis 5, **dadurch gekennzeichnet, dass** die Zuflussleitung und/oder die Druckleitung Kapillare sind.

7. Set umfassend eine Messvorrichtung nach einem der Ansprüche 1 bis 6 und ein Stillhütchen, welches einen Rand zur Anlage an einer Mutterbrust und einen im wesentlichen kegelförmigen Saugnippel aufweist, der dem Schutz der Brustwarze der Mutterbrust dient und in angelegter Position einen Hohlraum zwischen Brustwarze und Stillhütchen begrenzt, **dadurch gekennzeichnet, dass** die Messvorrichtung in dem Hohlraum angeordnet ist und lösbar mit dem Stillhütchen verbunden ist.

8. Set nach Anspruch 7, **dadurch gekennzeichnet, dass** die Messvorrichtung eine gewölbte Oberseite und eine Unterseite aufweist, wobei die Oberseite an einer Innenseite des Saugnippels anliegt und die Unterseite zum verbleibenden Hohlraum zeigt.

9. Set nach Anspruch 8, **dadurch gekennzeichnet, dass** die Oberseite der Messvorrichtung die Messkammer begrenzt und der Saugnippel an einem Anlagebereich, an der die Oberseite der Messvorrichtung anliegt, eine Skala für den in die Messkammer gelangten Messstrom aufweist.

10. Set nach einem der Ansprüche 7 bis 9, **dadurch gekennzeichnet, dass** der Rand des Stillhütchens eine Markierung zur richtigen Ausrichtung des Sets trägt.

11. Set nach einem der Ansprüche 9 oder 10, **dadurch gekennzeichnet, dass** an dem Anlagebereich des Saugnippels wenigstens eine rohrförmige Leitung vorgesehen ist, die sich vom Hohlraum durch den Saugnippel nach außen zu einer Spitze des Saugnippels erstreckt und in Verbindung zur Druckleitung der Messvorrichtung steht.

12. Set nach Anspruch 11, **dadurch gekennzeichnet, dass** an der Oberseite wenigstens eine nutförmige Vertiefung zur Aufnahme der rohrförmigen Leitung vorgesehen ist.

13. Set nach Anspruch 11 oder 12, **dadurch gekennzeichnet, dass** sich in die Vertiefung ein Rohrende der Druckleitung erstreckt, wobei die Messvorrichtung durch Stecken des Rohrendes in die rohrförmige Leitung an dem Stillhütchen befestigbar ist.

14. Set nach einem der Ansprüche 1 bis 10, **dadurch gekennzeichnet, dass** die Messvorrichtung einen Anströmkanal aufweist, welcher durch einen Mittelsteg geteilt ist, in dem die Zuflussleitung eingearbeitet ist.

15. Verfahren zum Messen eines Fluidstroms mit einer Messvorrichtung nach einem der Ansprüche 1 bis 6 oder mit einem Set nach einem der Ansprüche 7 bis 14, **dadurch gekennzeichnet, dass** die Messvorrichtung als Einwegartikel eingesetzt wird.
